# EUROPEAN PATENT APPLICATION

(11) **EP 4 730 171 A1**
(43) Date of publication of application: **22.04.2026**
(21) Application number: 24823221.7
(22) Date of filing: 28.05.2024
(51) Int. Cl.: G06F 21/35, A61B 5/00, A61B 5/0215, G06F 21/31, G06F 21/32, G06F 21/45, G06F 21/34, H04L 9/40, H04W 12/06

(54) **TERMINAL DEVICE AND METHOD**

(30) Priority: 15.06.2023 JP 2023098715
(71) Applicant: Sony Group Corporation, Tokyo 108-0075 (JP)
(72) Inventor: NARUSE, Tetsuya, Tokyo 108-0075 (JP)
(74) Representative: 2SPL Patentanwälte PartG mbB
(86) International application number: PCT/JP2024/019516
(87) International publication number: WO 2024/257601

(57) **Abstract**

A terminal device according to the present disclosure includes an authentication unit, a communication unit, a detection unit, and a stop processing unit. The authentication unit executes authentication processing for communicating with another device. The communication unit communicates with the other device when the authentication is successful. The detection unit detects removal of the terminal device, inserted into the body, to the outside of the body. The stop processing unit stops the authentication processing by the authentication unit when the removal to the outside of the body is detected.

## Description

### Technical Field

The present disclosure relates to a terminal device and a method.

### Background Art

In recent years, a technique of embedding a medical instrument, a microchip, or the like in a body in order to perform treatment, personal identification, or the like is known. In addition, for example, a technique is spreading of performing electronic payment or the like using an RFID tag embedded in a body.

### Citation List

### Patent Literature

PTL 1: JP 2023-18683 A
PTL 2: JP 2014-179105 A

### Summary

### Technical Problem

In electronic payment and personal identification using an RFID tag, impersonation is prevented by performing authentication using identification information stored in the RFID tag.

In the related art, it is assumed that the RFID tag is used by being inserted into the body of a person, and a case where the RFID tag is pulled out of the body of the person is not considered. It is desired to prevent unauthorized use of a terminal device (for example, an RFID tag) used by being inserted into the body even when the terminal device is removed from the body of a user.

Here, the present disclosure provides a mechanism capable of preventing unauthorized use of a terminal device that is inserted into a body and used.

The above problem or object is merely one of a plurality of problems or objects that can be solved or achieved by a plurality of embodiments disclosed in the present specification.

### Solution to Problem

A terminal device according to the present disclosure includes an authentication unit, a communication unit, a detection unit, and a stop processing unit. The authentication unit executes authentication processing for communicating with another device. The communication unit communicates with the other device when the authentication processing is successful. The detection unit detects removal, to the outside of a body, of the terminal device inserted into the body. The stop processing unit stops the authentication processing by the authentication unit when the removal to the outside of the body is detected.

### Brief Description of Drawings

[Fig. 1]

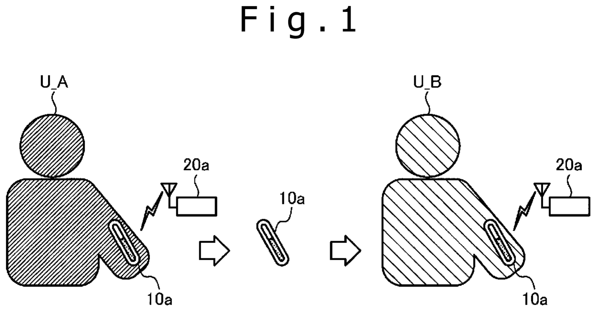

   Fig. 1 is a diagram illustrating an example of a method of using a terminal device.
[Fig. 2]

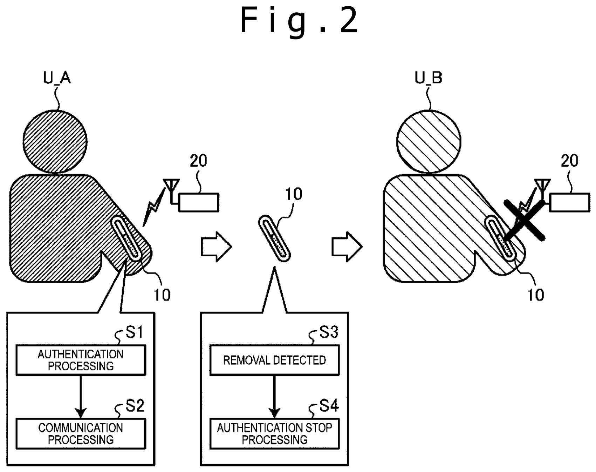

   Fig. 2 is a diagram illustrating an example of stop processing according to the proposed technology of the present disclosure.
[Fig. 3]

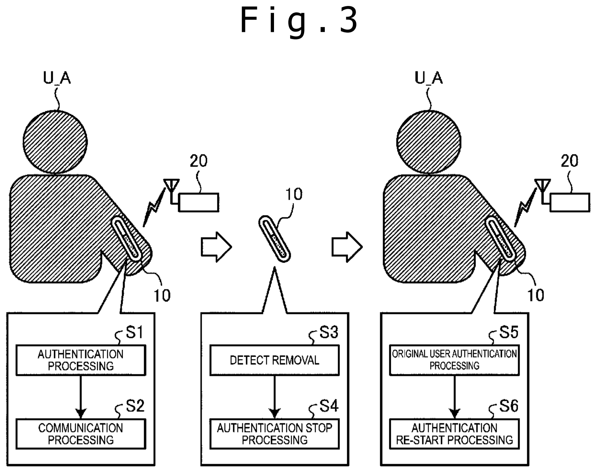

   Fig. 3 is a diagram illustrating an example of re-start processing according to the proposed technology of the present disclosure.
[Fig. 4]

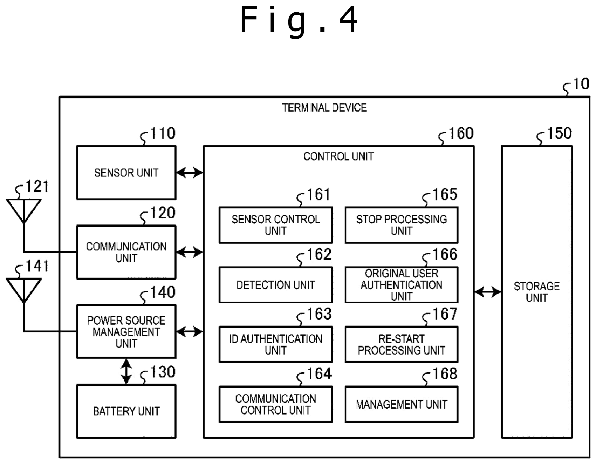

   Fig. 4 is a block diagram illustrating a functional configuration example of a terminal device according to an embodiment of the present disclosure.
[Fig. 5]

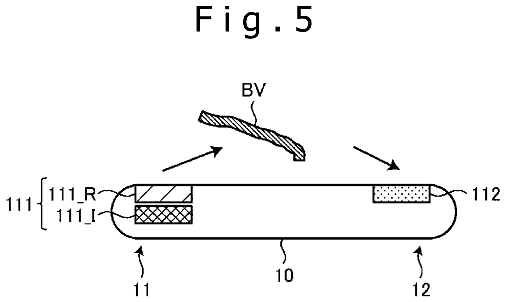

   Fig. 5 is a diagram illustrating an example of a PPG sensor according to the embodiment of the present disclosure.
[Fig. 6]

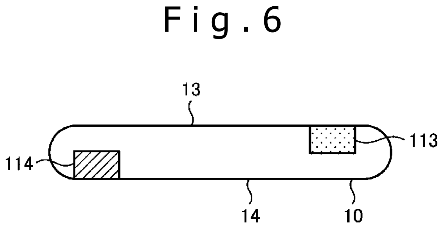

   Fig. 6 is a diagram illustrating an example of an ECG sensor according to the embodiment of the present disclosure.
[Fig. 7]

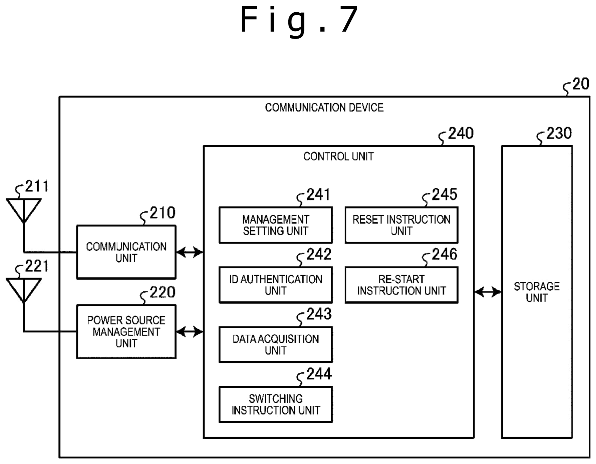

   Fig. 7 is a block diagram illustrating a functional configuration example of a communication device according to the embodiment of the present disclosure.
[Fig. 8]

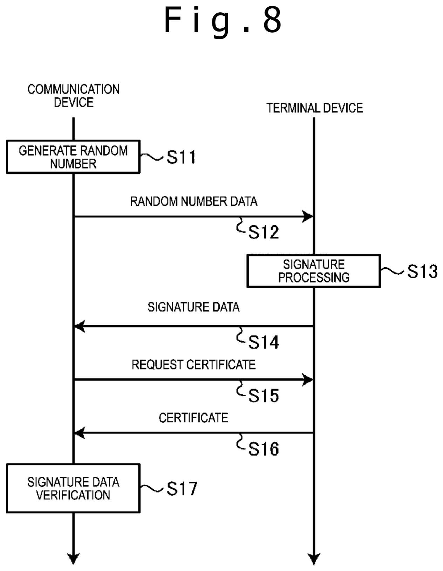

   Fig. 8 is a sequence diagram illustrating an example of a flow of authentication processing according to the embodiment of the present disclosure.
[Fig. 9]

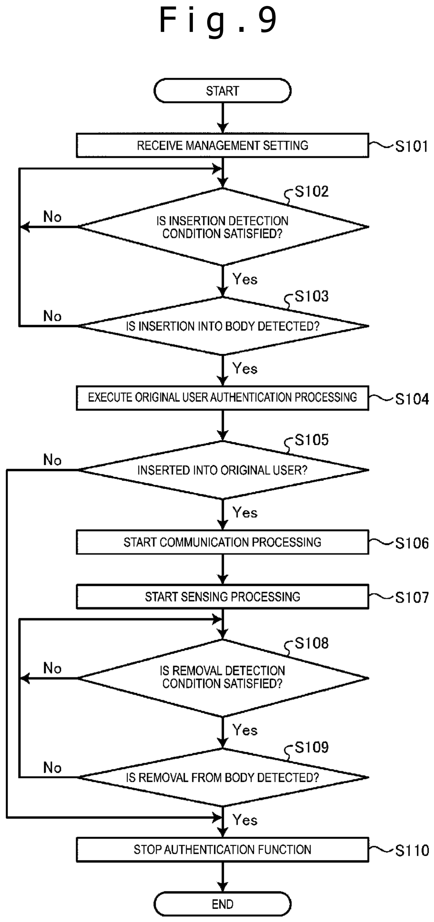

   Fig. 9 is a flowchart illustrating an example of a flow of first processing according to the embodiment of the present disclosure.
[Fig. 10]

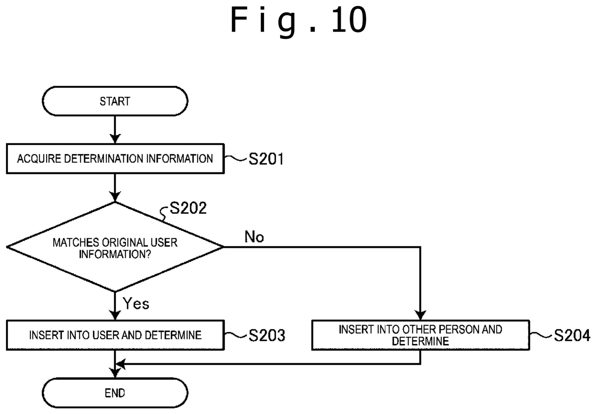

   Fig. 10 is a flowchart illustrating an example of a flow of original user authentication processing according to the embodiment of the present disclosure.
[Fig. 11]

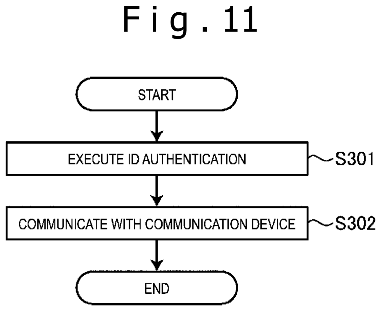

   Fig. 11 is a flowchart illustrating an example of a flow of communication processing according to the embodiment of the present disclosure.
[Fig. 12]

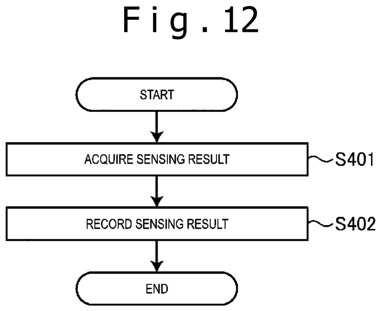

   Fig. 12 is a flowchart illustrating an example of a flow of sensing processing according to the embodiment of the present disclosure.
[Fig. 13]

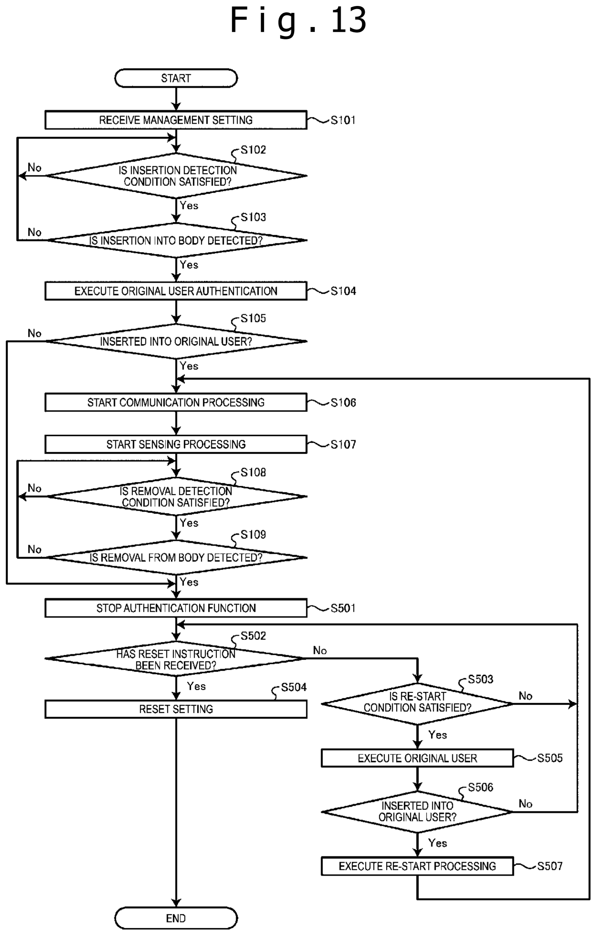

   Fig. 13 is a flowchart illustrating an example of a flow of second processing according to the embodiment of the present disclosure.

### Description of Embodiments

Hereinafter, embodiments of the present disclosure will be described in detail with reference to the accompanying drawings. Note that, in the present specification and the drawings, components having substantially the same functional configuration are denoted by the same reference numerals, and a redundant description thereof will be omitted.

Further, in the present specification and the drawings, similar components of the embodiments may be distinguished from each other by adding at least one of a different alphabetic character or number after the same reference numeral. However, when it is not necessary to particularly distinguish between each of the similar components, only the same reference numeral is assigned.

One or more embodiments (including examples, modifications, and application examples) described below can be implemented independently. On the other hand, at least some of the plurality of embodiments described below may be implemented in combination with at least a part of another of the embodiments as appropriate. The embodiments may include novel features that are different from each other. Therefore, the plurality of embodiments can contribute to solving different objects or problems and can achieve different effects.

### 1. Introduction

### 1.1 Problem

As described above, a technique is known for performing personal identification, electronic payment, or the like using a microchip inserted into a body. There is a risk of fraud by removing the microchip from the body of the person and reinserting it into the body of another person, for example.

Fig. 1 is a diagram illustrating an example of a method of using a terminal device 10a. As illustrated in the left diagram in Fig. 1, the terminal device 10a is inserted into the body (in Fig. 1, the arm, the back of the hand, or the like) of a user U_A. The terminal device 10a communicates with a communication device 20a located outside the body of the user U_A. Thus, the user U_A performs electronic payment, personal identification, and the like using the terminal device 10a.

Here, as illustrated in the center diagram in Fig. 1, it is assumed that the terminal device 10a is extracted from the body of the user U_A. The terminal device 10a does not detect that the terminal device 10a has been removed. Thus, as illustrated in the right diagram in Fig. 1, the terminal device 10a can communicate with the communication device 20a even when the terminal device 10a is inserted into a user U_B different from the user U_A.

In this way, the user U_B can impersonate the user U_A by removing the terminal device 10a from the body and inserting the terminal device 10a into the user U_B. It is desired to prevent such unauthorized use of the terminal device 10a.

### 1.2 Overview of Proposed Technology

Here, the terminal device according to the proposed technology of the present disclosure detects that the terminal device is inserted into the body, and that the terminal device has been removed from the body. When the terminal device has been removed from the body, the terminal device does not permit use of the terminal device. For example, when the terminal device performs authentication processing for communicating with the communication device, the terminal device is set to be in a state in which the terminal device cannot be used, by disabling the authentication processing.

Fig. 2 is a diagram illustrating an example of stop processing according to the proposed technology of the present disclosure. The stop processing is executed by a terminal device 10. The terminal device 10 is, for example, an implantable device that communicates with a communication device 20 in a state of being inserted into the body of the user U_A.

As illustrated in the left diagram in Fig. 2, the terminal device 10 performs wireless communication with the communication device 20. When the terminal device 10 is equipped with a radio frequency identifier (RFID) tag, the communication device 20 has a function of an IC chip reader, an RFID reader, or the like, for example.

The terminal device 10 first executes authentication processing prior to the wireless communication with the communication device 20 (step S1). For example, the terminal device 10 executes the authentication processing based on a public key encryption scheme. Alternatively, the terminal device 10 may execute the authentication processing with the communication device 20 using ID (device identification information) for identifying the terminal device 10. The ID for identifying the terminal device 10 is, for example, a device-specific ID assigned to each of the terminal devices 10.

Upon completion of the authentication processing with the communication device 20, the terminal device 10 executes communication processing with the communication device 20 (step S2). Through this communication processing, the terminal device 10 performs processing such as electronic payment, user identification, and transmission and reception of sensing data with the communication device 20.

Here, as illustrated in the middle diagram in Fig. 2, it is assumed that the terminal device 10 is removed from the user U_A. In this case, the terminal device 10 detects the removal of the terminal device 10 (step S3). The terminal device 10 detects the removal in accordance with a sensing result of a sensor mounted on the terminal device 10, for example.

The terminal device 10 that has detected the removal of the terminal device 10 executes authentication stop processing (step S4). The authentication stop processing is processing that causes the authentication processing at step S1 to not be performed. For example, the terminal device 10 may stop a CPU, or may stop the communication processing so that the authentication processing cannot be performed. Alternatively, the terminal device 10 may be configured not to perform the authentication processing by destroying a circuit or the like mounted on the terminal device 10.

As illustrated in the right diagram in Fig. 2, it is assumed that the terminal device 10 is inserted into the user U_B different from the user U_A. In this case, since the terminal device 10 inserted into the user U_B is executing the authentication stop processing, the terminal device 10 cannot execute the authentication processing with the communication device 20 and cannot communicate with the communication device 20.

Accordingly, the terminal device 10 can prevent the unauthorized use by the user U_B.

Here, in the authentication stop processing, when the authentication processing is disabled by stopping the CPU or by stopping the communication processing, the terminal device 10 can resume the authentication processing by resuming operation of the CPU or the communication processing.

Fig. 3 is a diagram illustrating an example of re-start processing according to the proposed technology of the present disclosure. The re-start processing is executed by the terminal device 10 subsequent to the execution of the stop processing. Processing illustrated in the left and middle diagrams in Fig. 3 is the same as the stop processing in Fig. 2, and a description thereof will thus be omitted.

As illustrated in the right diagram in Fig. 3, the terminal device 10 re-inserted into the body executes original user authentication processing (step S5). For example, the terminal device 10 confirms whether or not the terminal device 10 has been re-inserted into the original user (here, the user U_A), depending on whether or not a predetermined operation has been performed.

Examples of the predetermined operation include a password input from the user U_A, or original user authentication using biological information (a fingerprint, pulse, or the like) of the user U_A. Alternatively, for example, the terminal device 10 may re-start the authentication processing by causing an environmental state (an electromagnetic field, temperature, pressure, or the like) of the terminal device 10 to be a specified state using a special jig.

When it is confirmed, by the original user authentication processing, that the terminal device 10 has been re-inserted into the original user, the terminal device 10 executes authentication re-start processing (step S6). In this way, the terminal device 10 re-starts the authentication processing.

In this manner, when the predetermined operation is performed, the terminal device 10 may determine that the terminal device 10 has been re-inserted into the correct user U_A and may re-start the authentication processing. The authentication stop processing performed in order to be able to re-start the authentication processing is also described as recoverable stop processing or reversible stop processing.

On the other hand, for example, when the authentication processing is disabled by destroying the circuit or the like mounted on the terminal device 10, the terminal device 10 cannot re-start the authentication processing. In this way, the authentication stop processing in which the authentication processing cannot be re-started, that is, in which the function of the terminal device 10 is disabled once the terminal device 10 is removed (one-time use property) is also described as unrecoverable stop processing or irreversible stop processing.

The unrecoverable stop processing includes processing that stops the authentication in terms of software, and processing that stops the authentication in terms of hardware. For example, in a case where the authentication is stopped so that the recovery is not possible in terms of the software, the terminal device 10 erases programs and data. Further, for example, in a case where the authentication is stopped so that the recovery is not possible in terms of the hardware, the terminal device 10 destroys at least a part of the circuit by, for example, causing a large current to flow. Alternatively, the terminal device 10 may stop the authentication processing by moving a state of hardware logic that performs the authentication processing to a state to which state machine transition is not possible.

Note that the authentication stop processing described above is an example. It is sufficient that the terminal device 10 operates so as to disable its own function in the case where the terminal device 10 detects the removal from the body. For example, the terminal device 10 may execute the authentication stop processing using a one-time programmable read-only memory (OneTimePROM) or the like.

The terminal device 10 may operate by switching between the recoverable stop processing and the unrecoverable stop processing. For example, the terminal device 10 may switch between the recoverable stop processing and the unrecoverable stop processing in accordance with an instruction from an external device (the user U_A or an administrator of the terminal device 10), or in accordance with a predetermined condition (for example, a number of times of use, a usage time period, an application, or the like).

By executing the unrecoverable stop processing when the removal is detected, the terminal device 10 can more reliably prevent the unauthorized use in which the user U_A is impersonated. This allows the terminal device 10 to have a one-time use property at a time of implantation.

By performing the recoverable stop processing when the removal is detected, the terminal device 10 can re-start its function when the terminal device 10 is re-inserted into the user U_A.

### 2. Device Configuration Example

### 2.1 Configuration Example of Terminal Device

Fig. 4 is a block diagram illustrating a functional configuration example of the terminal device 10 according to an embodiment of the present disclosure. Note that Fig. 4 conceptually illustrates the functions of the terminal device 10, and various aspects may be adopted depending on the embodiment.

The terminal device 10 in Fig. 4 includes a sensor unit 110, a communication unit 120, a battery unit 130, a power source management unit 140, a storage unit 150, and a control unit 160.

### Sensor Unit 110

The sensor unit 110 senses a state surrounding the terminal device 10. The sensor unit 110 performs sensing in accordance with control of the control unit 160.

The sensor unit 110 can sense biological information of the user U_A in the state in which the terminal device 10 is inserted into the body of the user U_A, for example.

The sensor unit 110 can include a biological information sensor such as a photoplethysmography (PPG) sensor (a pulse sensor), an electrocardiogram (ECG) sensor, a temperature sensor, a blood glucose sensor, a blood pressure sensor, and the like.

The terminal device 10 according to the present embodiment is inserted into the body. Therefore, since the biological information sensor of the sensor unit 110 performs sensing inside the body, sensor unit 110 is less likely to be affected by noise and can perform sensing with higher accuracy. Since the biological information sensor of the sensor unit 110 has high accuracy, that is, a high sensing efficiency, power consumption can be suppressed to be low.

### PPG Sensor

The PPG sensor is a biological information sensor that detects a pulse wave signal of the user U_A. Here, the pulse wave signal is a waveform due to pulsation of the artery that appears at the body surface or the like when blood is delivered to the whole body through an artery, and a change in pressure occurs at the inner wall of the artery due to contraction of the muscle of the heart at a constant rhythm (the pulse, and note that the number of pulsations in the heart per unit time is referred to as a heart rate).

In order to acquire the pulse wave signal, the PPG sensor irradiates a blood vessel in a measurement site of the user with light and detects light scattered by a substance moving in the blood vessel of the target user or in stationary biological tissue.

Since the irradiated light is absorbed by red blood cells in the blood vessel, the amount of light absorbed is proportional to the amount of blood flowing through the blood vessel in the measurement site. Therefore, by detecting the intensity of the scattered light, the PPG sensor can ascertain a change in the amount of the blood flowing. Further, the PPG sensor can detect the pulse waveform, that is, the pulse wave signal from the change in the blood flow rate. Such a method is called the photoplethysmography (PPG) method.

Fig. 5 is a diagram illustrating an example of a PPG sensor according to the embodiment of the present disclosure. The PPG sensor includes a light source unit 111 (an example of a first sensor) that irradiates a blood vessel BV with light, and a light receiving unit 112 (an example of a second sensor) that receives light scattered by the blood vessel. In the example in Fig. 5, the PPG sensor includes a red light source unit 111_R that emits red light and an infrared light source unit 111_I that emits infrared light.

As illustrated in Fig. 5, the light source unit 111 is disposed at one end portion 11 of the terminal device 10. The light receiving unit 112 is disposed at another end portion 12 of the terminal device 10. The one end portion 11 and the other end portion 12 of the terminal device 10 are end portions facing each other.

The terminal device 10 is considered to be small because it is used by being inserted into the body, such as under the skin. For example, the terminal device 10 may have a shape such as a cylindrical shape or a flat plate shape. As described above, the shape of the terminal device 10, which is an implantable device, may be limited.

In this way, even when the shape of the terminal device 10 is limited, for example, to the cylindrical shape, the flat plate shape, or the like, the light source unit 111 and the light receiving unit 112 are arranged at both the ends of the terminal device 10, and thus the PPG sensor of the sensor unit 110 can suppress crosstalk between the light source unit 111 and the light receiving unit 112. Accordingly, the PPG sensor of the sensor unit 110 can sense the biological information with higher accuracy.

### ECG Sensor

The ECG sensor is a sensor that detects an electrocardiogram of the user U_A. The ECG sensor detects the electrocardiogram of the user U_A via a plurality of electrodes. The ECG sensor detects a signal indicating a heartbeat, using a potential difference between the plurality of electrodes.

Fig. 6 is a diagram illustrating an example of the ECG sensor according to the embodiment of the present disclosure. The ECG sensor illustrated in Fig. 6 includes electrodes 113 and 114 (examples of a third sensor and a fourth sensor). Although the ECG sensor in Fig. 6 includes the two electrodes 113 and 114, the number of electrodes included in the ECG sensor is not limited to two, and may be three or more.

For example, the electrode 113 is a potential electrode, and the electrode 114 is a ground (GND) electrode. The ECG sensor detects the electrocardiogram of the user U_A in accordance with the potential difference between the electrodes 113 and 114.

As illustrated in Fig. 6, the electrode 113 is disposed at a first surface 13 of the terminal device 10. The electrode 114 is disposed at a second surface 14 of the terminal device 10. The first surface 13 and the second surface 14 of the terminal device 10 face each other. The electrodes 113 and 114 are disposed respectively at both the end portions of the terminal device 10.

In this manner, by disposing the electrodes 113 and 114 at the first surface 13 and the second surface 14, respectively, of the terminal device 10, the electrodes 113 and 114 of the ECG sensor can be separated from each other. Accordingly, even when the shape of the terminal device 10 is limited, for example, to the cylindrical shape, the flat plate shape, or the like, the ECG sensor can further improve the detection accuracy of the electrocardiogram.

In addition to the above-described biological information sensor, the sensor unit 110 includes a detection sensor that detects at least one of the insertion or the removal of the terminal device 10. Examples of the detection sensor include a capacitance sensor, an impedance sensor, a temperature sensor, a humidity sensor, a pressure sensor, an ultrasonic sensor, and a strain sensor.

The sensor unit 110 outputs the sensing results (the sensing result of the biological information sensor and the sensing result of the detection sensor) to the control unit 160.

### Communication Unit 120

The communication unit 120 is a wireless communication processing unit that communicates with the communication device 20. The communication unit 120 may operate by receiving power supply from the communication device 20 (passive type), or may operate by receiving power supply from the battery unit 130 (active type). The communication unit 120 can include a circuit, such as a rectifier.

The communication unit 120 includes an antenna 121. The communication unit 120 can perform short-range wireless communication, such as near field communication (NFC) or Bluetooth (registered trademark). In a case where the communication unit 120 supports a plurality of wireless access systems, each of the units constituting the communication unit 120 may be individually configured for each of the wireless access systems.

### Battery Unit 130

The battery unit 130 supplies power to each of the units of the terminal device 10. The battery unit 130 performs charging or discharging in accordance with the control of the power source management unit 140. The terminal device 10 may include a power supply for driving the terminal device 10, and may include a capacitor or the like instead of the battery unit 130.

### Power Source Management Unit 140

The power source management unit 140 manages the charging of the battery unit 130 or the discharging from the battery unit 130. For example, in a case where the power source management unit 140 receives power supplied from an external device (not illustrated) via an antenna 141, the power source management unit 140 charges the battery unit 130 using the supplied power. The power source management unit 140 supplies the power of the battery unit 130 to each of the units of the terminal device 10.

Note that, although Fig. 4 illustrates a case where the antenna 121 that performs communication and the antenna 141 that performs wireless charging are different, the terminal device 10 may perform both communication and wireless charging with one antenna.

### Storage Unit 150

The storage unit 150 is a storage device capable of reading and writing data, such as a dynamic random access memory (DRAM), a static random access memory (SRAM), a flash memory, or the like. The storage unit 150 stores, for example, a sensing result by the sensor unit 110.

### Control Unit 160

The control unit 160 is a controller that controls each of the units of the terminal device 10. The control unit 160 may be realized by a processor, such as a central processing unit (CPU) or a micro-processing unit (MPU). Specifically, the control unit 160 may be realized by a processor executing various programs stored in a storage device inside the terminal device 10 using a random access memory (RAM) or the like as a work area.

The control unit 160 may be realized by an integrated circuit, such as an application-specific integrated circuit (ASIC), a field programmable gate array (FPGA), or the like. The control unit 160 may be realized by a graphics processing unit (GPU). Any of the CPU, the MPU, the ASIC, the FPGA, and the GPU can be regarded as a controller. The control unit 160 may be configured by a plurality of physically separated objects. For example, the control unit 160 may be configured by a plurality of semiconductor chips.

The control unit 160 includes a sensor control unit 161, a detection unit 162, an ID authentication unit 163, a communication control unit 164, a stop processing unit 165, an original user authentication unit 166, a re-start processing unit 167, and a management unit 168.

Each of the blocks (the sensor control unit 161 to the management unit 168) constituting the control unit 160 is a functional block indicating a function of the control unit 160. These functional blocks may be software blocks or hardware blocks.

For example, each of the above-described functional blocks may be a single software module realized by software (including a microprogram) or may be a single circuit block on a semiconductor chip (die). Of course, each of the functional blocks may be a single processor or a single integrated circuit. The control unit 160 may be configured by functional units different from the above-described functional blocks. The method of configuring the functional blocks is arbitrary.

### Sensor Control Unit 161

The sensor control unit 161 controls the sensor unit 110 to acquire the sensing result. For example, when the terminal device 10 receives a management setting from the communication device 20 and subsequently an insertion detection condition is satisfied, the sensor control unit 161 controls the detection sensor of the sensor unit 110 to acquire the sensing result. The sensor control unit 161 outputs the acquired sensing result to the detection unit 162.

The communication device 20 transmits the management setting as preparation for inserting the terminal device 10 into the body of the user U_A, for example. Therefore, when the terminal device 10 receives the management setting, the terminal device 10 waits for the insertion into the body, as the insertion into the body of the user U_A. Specifically, when the insertion detection condition is satisfied, the sensor control unit 161 controls the sensor unit 110 to perform sensing for detecting the insertion of the terminal device 10 into the body.

Here, the insertion detection condition may include a time period elapsed from when the management setting is received, whether the terminal device 10 has moved, or the like. The sensor control unit 161 controls the sensor unit 110 to perform the sensing for detecting the insertion of the terminal device 10 into the body after a predetermined time period has elapsed from when the management setting is received, or when the terminal device 10 has moved.

When the terminal device 10 is inserted into the body, the sensor control unit 161 controls the biological information sensor of the sensor unit 110 at a predetermined cycle to acquire the sensing result. The sensor control unit 161 stores the acquired sensing result in the storage unit 150.

When the detection unit 162 detects the insertion into the body, the sensor control unit 161 controls the biological information sensor of the sensor unit 110 to acquire the sensing result. The sensor control unit 161 outputs the acquired sensing result to the original user authentication unit 166.

### Detection Unit 162

The detection unit 162 detects at least one of the insertion of the terminal device 10 into the body or the removal of the terminal device 10 from the body, in accordance with the sensing result of the sensor unit 110. For example, when the sensing result of the temperature sensor of the sensor unit 110 is used, the detection unit 162 detects the insertion/removal in accordance with a change in the temperature (body temperature/environmental temperature) detected by the temperature sensor. In this manner, the detection unit 162 detects the insertion/removal in accordance with the change in the sensing result of the sensor unit 110.

### ID Authentication Unit 163

The ID authentication unit 163 performs the authentication processing with the communication device 20 prior to the communication processing using the communication unit 120. The ID authentication unit 163 performs the authentication processing with the communication unit 120 using a public key encryption technique, for example. Alternatively, the ID authentication unit 163 may execute the authentication processing with the communication device 20 using the identification information (device ID) of the terminal device 10 stored in advance. An example of the authentication processing will be described later with reference to Fig. 8.

The ID authentication unit 163 acquires a result of the authentication processing (authentication success/failure) from the communication device 20, and outputs the result to the communication control unit 164.

### Communication Control Unit 164

The communication control unit 164 performs wireless communication by controlling the communication unit 120, for example. For example, when the authentication is successful as a result of the authentication processing by the ID authentication unit 163, the communication control unit 164 communicates with a communication counterpart (the communication device 20, for example).

For example, the communication control unit 164 transmits the sensing result (for example, the biological information or the like) of the sensor unit 110 to the communication device 20. The communication control unit 164 can execute electronic payment processing and the like with the communication device 20. The communication control unit 164 acquires control information, such as the management setting from the communication device 20.

### Stop Processing Unit 165

When the detection unit 162 detects the removal of the terminal device 10, the stop processing unit 165 executes the authentication stop processing. As described above, the authentication stop processing includes the recoverable stop processing that can recover the function of the terminal device 10, and the unrecoverable stop processing that cannot recover the function of the terminal device 10 (cannot re-start the authentication).

### Recoverable Stop Processing

An example of the recoverable stop processing is stopping the operation of the control unit 160. For example, the stop processing unit 165 causes the power source management unit 140 to stop the supply of power to the control unit 160. Alternatively, the stop processing unit 165 instructs the control unit 160 to stop operation. The stop processing unit 165 may be configured to not stop some of the functions (the detection unit 162, the original user authentication unit 166, and the re-start processing unit 167 to be described later) of the control unit 160, which are to be used for the recovery.

Another example of the recoverable stop processing is stopping the operation of the communication unit 120 (or the communication control unit 164). For example, the stop processing unit 165 causes the power source management unit 140 to stop the supply of power to the communication unit 120 (or the communication control unit 164). Alternatively, the stop processing unit 165 instructs the communication unit 120 (or the communication control unit 164) to stop operation.

Another example of the recoverable stop processing is processing that prohibits reading of a program, data, or the like from the storage unit 150. For example, the stop processing unit 165 makes it impossible to read a program for performing the authentication processing, identification information of the terminal device 10, and the like from the storage unit 150 that stores the program, the identification information, and the like. Alternatively, the stop processing unit 165 may instruct the storage unit 150 to stop the reading.

### Unrecoverable Stop Processing

An example of the unrecoverable stop processing is processing that stops the authentication processing in terms of the software. In order to stop the authentication processing in terms of the software, the stop processing unit 165 deletes a program for operating the terminal device 10, such as a program related to the authentication processing, a program for the communication processing, or the like. Alternatively, the stop processing unit 165 may delete data used for the authentication processing, such as the identification information of the terminal device 10, or data used how to operate the terminal device 10, such as data used for the communication processing.

An example of the unrecoverable stop processing is processing of stopping the authentication processing in terms of the hardware. In order to stop the authentication process in terms of the hardware, the stop processing unit 165 physically destroys a circuit or the like, for example. The stop processing unit 165 destroys the circuit by burning off the wiring of the circuit by applying a high voltage. Alternatively, the stop processing unit 165 may break down an insulating film of the circuit by applying the high voltage.

The stop processing unit 165 can also disable subsequent operations (e.g., operations such as the authentication processing and the communication processing) by moving to a state in which state machine transition by the hardware logic is not possible.

The stop processing unit 165 can stop the authentication processing in terms of the hardware by writing a Disable flag in the OneTimePROM so that the OneTimePROM cannot be subsequently changed. A method of writing the Disable flag includes the method of burning off the wiring and the method of destroying the insulating film, using high voltage, for example.

Alternatively, an electrically erasable programmable read only memory (EEPROM) in which data can be electrically written to a read only memory (ROM) may be used as the storage unit 150. In this case, the stop processing unit 165 stops the authentication processing by releasing the Disable flag using a high voltage that is not normally used.

In this way, the stop processing unit 165 executes the unrecoverable stop processing, and thus, once the terminal device 10 is removed from the body, the terminal device 10 cannot be re-used. Since the terminal device 10 is used by being inserted into the body, the number of times of use may be limited to one time in consideration of hygiene.

By configuring a housing of the terminal device 10 using a sterilizable material such as glass, the terminal device 10 may be re-inserted into the body of the user U_A and used. In such a case, the stop processing unit 165 performs the recoverable stop processing.

Alternatively, as to be described later, the terminal device 10 may be inserted and used by the user U_B different from the user U_A by completely resetting the state of the terminal device 10. In this way, in a case where the terminal device 10 is re-used by a different user U_B, it is necessary to reliably perform a process for re-use (for example, a sterilization process, a reset process, or the like), such as dedicated re-use processing performed on the terminal device 10 by a specialized processing company or the like.

The stop processing unit 165 prevents the personal information related to the user U_A stored in the storage unit 150 from being read from the storage unit 150. Examples of the personal information include the sensing result by the sensor unit 110, a communication history with the communication device 20 such as a payment history, and the like.

In this way, by not allowing access to the storage unit 150, the stop processing unit 165 prevents the unauthorized use of the personal information.

When the removal of the terminal device 10 is detected, the security of the terminal device 10 can be further enhanced by the stop processing unit 165 executing the authentication stop processing or the like.

### Original User Authentication Unit 166

The original user authentication unit 166 (an example of a wearable authentication unit) executes the original user authentication processing of verifying that an insertion destination of the terminal device 10 is the user U_A. The original user authentication unit 166 executes the original user authentication processing when the detection unit 162 detects the insertion of the terminal device 10. Alternatively, the original user authentication unit 166 may execute the original user authentication processing when the authentication processing is executed by the ID authentication unit 163.

The original user authentication unit 166 executes the original user authentication processing by comparing identification information for identifying the original user (hereinafter also referred to as original user information or original user ID) with determination information.

The original user information is information determined in advance by the user U_A or the like, such as a password, or is feature information of the user U_A. The feature information of the user U_A may include, for example, information relating to a feature amount extracted from the biological information of the user U_A. Alternatively, the original user information may be information that designates a specific environmental state.

The feature information of the user U_A may be acquired from the communication device 20, or may be generated by the original user authentication unit 166 on the basis of the sensing result by the sensor unit 110.

The original user authentication unit 166 acquires the determination information for performing the original user authentication processing from the communication device 20. Alternatively, the original user authentication unit 166 may acquire the determination information by generating the determination information in accordance with the sensing result by the sensor unit 110.

For example, when the original user information is a password, the original user authentication unit 166 acquires the password as the determination information from the communication device 20.

It is assumed that the original user information is the feature information extracted from the biological information (for example, a fingerprint, an iris, a face, or the like) using a sensor of the communication device 20. In this case, the original user authentication unit 166 acquires the biological information of the user U_A or information extracted from the biological information from the communication device 20, as the determination information.

In this way, when the feature information is acquired using a sensor or the like of an external device (the communication device 20, for example), a location where the original user authentication processing is executed is limited to a location where there is an external device capable of acquiring the feature information. Therefore, the terminal device 10 can further improve the security of the original user authentication processing.

It is assumed that the original user information is the feature information generated by the original user authentication unit 166 based on the biological information (pulse, heartbeat, blood pressure, blood glucose, and the like) which is the sensing result by the sensor unit 110. In this case, the original user authentication unit 166 acquires the sensing result from the sensor unit 110, and generates the determination information (feature information) from the sensing result. The pulse and the heartbeat can be acquired from the PPG, ECG sensor or the like, for example. The blood pressure can be acquired from a pressure sensor or a blood flow rate sensor. The blood glucose may be obtained from a sensor that can check body fluids.

In this way, by generating the original user information from the sensor unit 110, the original user authentication unit 166 can execute the original user authentication processing without communicating with an external device (for example, the communication device 20). This enables the original user authentication unit 166 to further prevent information leakage at the time of biometric authentication.

Note that the terminal device 10 may acquire the original user information from the communication device 20, even when the original user information is generated from the biological information that can be acquired from the sensing result by the sensor unit 110, such as the pulse, the heartbeat, the blood pressure, or the blood glucose level. In this case, the terminal device 10 acquires the generated information acquired by the communication device 20, the external device, or the like, or acquires the feature information extracted from the generated information, as the original user information via the communication device 20.

In this manner, by acquiring the original user information generated from the biological information from the communication device 20, the original user authentication unit 166 does not need to generate the original user information from the sensing result of the sensor unit 110. Thus, for example, the original user authentication processing can be executed even when the original user authentication unit 166 cannot generate the original user information, such as a timing at which the terminal device 10 is inserted into the body for the first time.

In a case where at least one of the original user information or the determination information is acquired via the communication device 20, communication is performed between the terminal device 10 and the communication device 20. It is desirable that an encrypted secure protocol be used for this communication. This makes it possible to further improve the security of the original user authentication processing.

Note that, although here the original user authentication unit 166 compares the original user information and the determination information, an external device (for example, the communication device 20) may perform the comparison. In this case, the communication device 20 holds the original user information in advance, and, based on the determination information acquired from the user U_A, compares the identification information and the determination information. The original user authentication unit 166 may acquire a comparison result from the communication device 20 and perform the original user authentication in accordance with the comparison result. In this way, at least a part of the original user authentication processing may be executed by an external device (the communication device 20, for example).

It is assumed that the original user information is information designating the specific environmental state (information relating to an electromagnetic field, temperature, pressure, and the like). In this case, the original user authentication unit 166 generates the determination information based on the sensing result acquired from the sensor unit 110. Note that the specific environmental state may be set using the special jig, for example. In this way, the user U_A can perform the original user authentication using a dedicated device.

### Re-start Processing Unit 167

When the detection unit 162 detects the insertion of the terminal device 10 in a state in which the authentication processing is stopped, the re-start processing unit 167 executes the authentication re-start processing of re-starting the authentication processing, in accordance with the result of the original user authentication by the original user authentication unit 166.

Alternatively, when the re-start processing unit 167 receives an instruction to execute the re-start processing from the communication device 20 (an example of the external device), the re-start processing unit 167 executes the authentication re-start processing for re-starting the authentication processing, in accordance with the result of the original user authentication by the original user authentication unit 166.

In this way, when some of the functions of the terminal device 10 (for example, the ID authentication function) are restricted, the re-start processing unit 167 executes processing that recovers the restricted function.

When the original user authentication is successful, the re-start processing unit 167 determines that the terminal device 10 has been re-inserted into the correct user U_A, and executes the authentication re-start processing that re-starts the authentication processing. When the original user authentication has failed, the re-start processing unit 167 does not execute the authentication re-start processing. Alternatively, when the original user authentication has failed, the re-start processing unit 167 may request the stop processing unit 165 to execute the unrecoverable stop processing, assuming that the terminal device 10 is inserted into the unauthorized user U_B.

As the authentication re-start processing, for example, the re-start processing unit 167 notifies the power source management unit 140 to supply power to the control unit 160 and the communication unit 120. Alternatively, the re-start processing unit 167 notifies the storage unit 150 to permit the reading.

### Management Unit 168

The management unit 168 manages the operations of the control unit 160. For example, the management unit 168 switches the authentication stop processing performed by the stop processing unit 165 between the recoverable stop processing and the unrecoverable stop processing, in accordance with the predetermined condition. For example, the management unit 168 switches between the recoverable stop processing and the unrecoverable stop processing in accordance with the instruction from the communication device 20, the usage time period of the terminal device 10, the number of times of use, the application, and the like.

The management unit 168 may execute reset processing of resetting the terminal device 10. The reset processing may be performed simultaneously with the unrecoverable stop processing or may be performed separately from the unrecoverable stop processing. The reset processing includes processing of deleting the identification information, the original user information, the personal information, and the like of the terminal device 10. Alternatively, the reset processing may include processing of deleting a program or the like executed by the terminal device 10.

The management unit 168 executes the reset processing in accordance with an instruction from the communication device 20. At this time, the management unit 168 can request the communication device 20 to perform authentication for executing the reset processing, such as the input of a password. This password may be a master password of a user having administrator authority.

### 2.2 Configuration Example of Communication Device

The communication device 20 (the example of the other device) is an information processing device that communicates with the terminal device 10. The communication device 20 may be an RFID tag reading device, a mobile terminal such as a smartphone or a tablet terminal, a PC, or the like.

Fig. 7 is a block diagram illustrating a functional configuration example of the communication device 20 according to the embodiment of the present disclosure. Note that Fig. 7 conceptually illustrates the functions of the communication device 20, and various aspects may be adopted depending on the embodiment.

The communication device 20 in Fig. 7 includes a communication unit 210, a power source management unit 220, a storage unit 230, and a control unit 240.

### Communication Unit 210

The communication unit 210 is a wireless communication processing unit that communicates with the terminal device 10. The communication unit 210 includes an antenna 211. The communication unit 210 can perform short-range wireless communication such as near field communication (NFC) or Bluetooth (registered trademark). In a case where the communication unit 210 supports a plurality of wireless access systems, each of the units constituting the communication unit 210 may be individually configured for each of the wireless access systems.

### Power Source Management Unit 220

The power source management unit 220 supplies power to the terminal device 10. The power source management unit 220 transmits power for charging the battery unit 130 of the terminal device 10 via an antenna 221.

Note that, although Fig. 7 illustrates a case where an antenna 211 that performs communication and the antenna 221 that performs wireless charging are different, the communication device 20 may perform both communication and wireless charging with one antenna.

### Storage Unit 230

The storage unit 230 is a storage device capable of reading and writing data, such as a DRAM, an SRAM, a flash memory, a hard disk, or the like. The storage unit 230 stores the sensing results and the like acquired from the terminal device 10, for example.

### Control Unit 240

The control unit 240 is a controller that controls each of the units of the communication device 20. The control unit 240 may be realized by a processor, such as a CPU or an MPU, for example. Specifically, the control unit 240 may be realized by a processor executing various programs stored in a storage device inside the communication device 20 using a random access memory (RAM) or the like as a work area.

The control unit 240 may be realized by an integrated circuit, such as an ASIC, an FPGA, or the like. The control unit 240 may be realized by a GPU. Any of the CPU, the MPU, the ASIC, the FPGA, and the GPU can be regarded as the controller. The control unit 240 may be configured by a plurality of physically separated objects. For example, the control unit 240 may be configured by a plurality of semiconductor chips.

The control unit 240 includes a management setting unit 241, an ID authentication unit 242, a transmission/reception unit 243, a switching instruction unit 244, a reset instruction unit 245, and a re-start instruction unit 246.

Each of the blocks (the management setting unit 241 to the re-start instruction unit 246) constituting the control unit 240 is a functional block indicating a function of the control unit 240. These functional blocks may be software blocks or hardware blocks.

For example, each of the above-described functional blocks may be a single software module realized by software (including a microprogram) or may be a single circuit block on a semiconductor chip (die). Of course, each of the functional blocks may be a single processor or a single integrated circuit. The control unit 160 may be configured by functional units different from the above-described functional blocks. The method of configuring the functional blocks is arbitrary.

### Management Setting Unit 241

The management setting unit 241 performs setting of the terminal device 10 by transmitting the management setting to the terminal device 10. The management setting unit 241 performs initial setting of the terminal device 10 by transmitting the management setting before the terminal device 10 is inserted into the body of the user U_A. For example, the management setting may include information for pairing with the terminal device 10, or the original user information, such as the password and the like.

In this way, pairing processing is executed between the terminal device 10 and the communication device 20, or the original user information is set, as necessary.

### ID Authentication Unit 242

The ID authentication unit 242 verifies whether the terminal device 10 is a correct communication counterpart, prior to the communication processing using the communication unit 210. The ID authentication unit 242 executes the authentication processing with the terminal device 10 using, for example, the identification information (device ID) of the terminal device 10 stored in advance.

### Transmission/Reception Unit 243

The transmission/reception unit 243 transmits and receives data to and from the terminal device 10 via the communication unit 210. For example, the transmission/reception unit 243 acquires the sensing result by the sensor unit 110 from the terminal device 10. Further, for example, the transmission/reception unit 243 acquires data used in processing performed with the terminal device 10, such as information necessary for electronic payment.

The transmission/reception unit 243 may transmit, to the terminal device 10, information related to a result of processing performed with the terminal device 10, such as balance information, a payment history, or the like. In this way, the transmission/reception unit 243 exchanges data with the terminal device 10.

### Switching Instruction Unit 244

The switching instruction unit 244 instructs the terminal device 10 to switch between the recoverable stop processing and the unrecoverable stop processing. The switching instruction unit 244 switches between the recoverable stop processing and the unrecoverable stop processing in accordance with an instruction from the administrator or the user U_A, or with the usage time period, the number of times of use, the application, and the like of the terminal device 10.

For example, when the usage time period exceeds a certain time period or when the number of times of use exceeds a certain number of times, the switching instruction unit 244 instructs the terminal device 10 to execute the unrecoverable stop processing. The switching instruction unit 244 instructs the terminal device 10 to execute the recoverable stop processing in the case of an experiment or trial use, and to switch to the unrecoverable stop processing in the case of actual use.

### Reset Instruction Unit 245

The reset instruction unit 245 instructs the terminal device 10 to execute the reset processing. The reset instruction unit 245 notifies the terminal device 10 to execute the reset processing in accordance with an instruction from the administrator or the user U_A, for example.

### Re-start Instruction Unit 246

The re-start instruction unit 246 instructs the terminal device 10 to execute the authentication re-start processing. The re-start instruction unit 246 notifies the terminal device 10 of a request to execute the authentication re-start processing in accordance with an instruction from the administrator or the user U_A, for example.

### 3. Information Processing Example

### 3.1 Authentication Processing Example

The authentication processing executed prior to communication when the terminal device 10 and the communication device 20 communicate with each other will be described. The authentication processing is executed in order for the terminal device 10 and the communication device 20 to communicate with each other.

Fig. 8 is a sequence diagram illustrating an example of a flow of the authentication processing according to the embodiment of the present disclosure. Here, an overview of the authentication processing using a public key encryption technique will be described.

As illustrated in Fig. 8, the communication device 20 generates a random number used for the authentication processing (step S11). The communication device 20 transmits random number data including the generated random number to the terminal device 10 (step S12).

The terminal device 10 that has received the random number data executes signature processing that generates signature data using the random number data and a private key (step S13). The private key is, for example, information related to the identification information (device ID) of the terminal device 10. The terminal device 10 generates the signature data by encrypting the random number data using the private key, for example.

The terminal device 10 transmits the generated signature data to the communication device 20 (step S14). The communication device 20 that has received the signature data requests the terminal device 10 to transmit a certificate including public key information related to a public key (step S15).

The terminal device 10 transmits the certificate including the public key information to the communication device 20 in response to the request (step S16). The communication device 20 that has received the certificate verifies the signature data (step S17). For example, the communication device 20 decrypts the signature data using the public key information included in the certificate. The communication device 20 verifies whether the terminal device 10 holds the assigned private key based on, for example, the decrypted data of the signature data.

When it is verified that the terminal device 10 holds the assigned private key, in other words, that the terminal device 10 is the correct communication counterpart, the communication device 20 determines that the authentication is successful. On the other hand, when it is verified that the terminal device 10 does not hold the assigned private key, in other words, that the terminal device 10 is not the correct communication counterpart, the communication device 20 determines that the authentication has failed.

The result of the authentication processing is notified from the communication device 20 to the terminal device 10, for example. After the authentication processing, for example, communication (not illustrated) is performed between the communication device 20 and the terminal device 10.

### 3.2 First Processing Example

First processing executed by the terminal device 10 when the authentication stop processing is the unrecoverable stop processing will be described.

Fig. 9 is a flowchart illustrating an example of a flow of first processing according to the embodiment of the present disclosure.

The terminal device 10 receives the management setting from the communication device 20 (step S101). Based on the management setting, the terminal device 10 can perform pairing processing with the communication device 20, and can set the original user information including the password, feature information, and the like.

Subsequently, the terminal device 10 determines whether or not the insertion detection condition is satisfied (step S102). The insertion detection condition includes the elapsed time from the reception of the management setting and movement of the terminal device 10. That is, the terminal device 10 determines that the insertion detection condition is satisfied when a predetermined time period has elapsed from when the management setting is received or when the terminal device 10 has moved by a predetermined value or more.

If the insertion detection condition is not satisfied (No at step S102), the terminal device 10 returns to step S102.

On the other hand, when the insertion detection condition is satisfied (Yes at step S102), the terminal device 10 determines whether or not the insertion into the body is detected (step S103). The terminal device 10 determines whether or not the terminal device 10 is inserted into the user U_A on the basis of the sensing result of the sensor unit 110.

If the insertion into the body is not detected (No at step S103), the terminal device 10 returns to step S102. Note that, although here the terminal device 10 returns to step S102, the terminal device 10 can return to step S103. Alternatively, the terminal device 10 may wait for a certain period of time to detect the insertion into the body again.

When the insertion into the body is detected (Yes at step S103), the terminal device 10 executes the original user authentication processing (step S104).

Here, the original user authentication processing will be described with reference to Fig. 10. Fig. 10 is a flowchart illustrating an example of a flow of the original user authentication processing according to the embodiment of the present disclosure.

The terminal device 10 acquires the determination information (step S201). The terminal device 10 acquires the determination information from the communication device 20 or from the sensing result by the sensor unit 110.

The terminal device 10 determines whether the acquired determination information matches the original user information held in advance (or acquired from the communication device 20) (step S202).

When the determination information and the original user information match (Yes at step S202), the terminal device 10 determines that the terminal device 10 is inserted into the user U_A (step S203). That is, the terminal device 10 determines that the original user authentication is successful.

On the other hand, when the determination information does not match the original user information (No at step S202), the terminal device 10 determines that the terminal device 10 is inserted into a person other than the user U_A (step S204). That is, the terminal device 10 determines that the original user authentication has failed.

When the determination information and the original user information include the feature amount of the biological information, the terminal device 10 determines whether or not the determination information and the original user information match each other according to whether or not a predetermined number or more of feature points (or feature amounts) match each other.

Referring back to Fig. 9, based on the result of the original user authentication processing, the terminal device 10 determines whether the terminal device 10 is inserted into the original user U_A (step S105).

When the original user authentication has failed and it is determined that the terminal device 10 is not inserted into the original user U_A, that is, the terminal device 10 is inserted into the user U_B other than the user U_A (No at step S105), the terminal device 10 advances to step S110.

On the other hand, when the original user authentication is successful, and it is determined that the terminal device 10 is inserted into the original user U_A (Yes at step S105), the terminal device 10 starts the communication processing (step S106) and starts sensing processing (step S107).

Here, the communication processing will be described with reference to Fig. 11. Fig. 11 is a flowchart illustrating an example of a flow of the communication processing according to the embodiment of the present disclosure.

The communication processing in Fig. 11 is executed by the terminal device 10 in accordance with a request from the communication device 20, for example. Alternatively, the communication processing may be executed when power is supplied from the communication device 20. Further, the communication processing may be executed at a constant cycle.

The terminal device 10 performs ID authentication (step S301). For example, the terminal device 10 performs the ID authentication by performing the authentication processing using the device ID.

When the ID authentication is successful, the terminal device 10 communicates with the communication device 20 (step S302).

Here, the sensing process will be described with reference to Fig. 12. Fig. 12 is a flowchart illustrating an example of a flow of the sensing processing according to the embodiment of the present disclosure.

The sensing processing in Fig. 12 is executed by the terminal device 10 at a constant cycle, for example. Alternatively, the sensing processing may be executed at the time of a request from the communication device 20.

The terminal device 10 acquires the sensing result from the sensor unit 110 (step S401). The terminal device 10 records the acquired sensing result (step S402). For example, the terminal device 10 stores the acquired sensing result in the storage unit 150. Alternatively, the terminal device 10 may notify the communication device 20 of the acquired sensing result.

The terminal device 10 executes the sensing processing, for example, at a constant cycle or based on a request from the communication device 20. This enables the terminal device 10 to perform the sensing with low power consumption.

Referring back to Fig. 9, the terminal device 10 determines whether or not a removal detection condition is satisfied (step S108). For example, the removal detection condition is an elapsed time period from the previous removal detection. For example, when a certain time period has elapsed from the previous removal detection, the terminal device 10 determines that the removal detection condition is satisfied.

If the removal detection condition is not satisfied (No at step S108), the terminal device 10 returns to step S108.

On the other hand, when the detection condition is satisfied (Yes at step S108), the terminal device 10 determines whether or not the removal from the body has been detected (Step S109). The terminal device 10 determines whether or not the terminal device 10 has been removed from the body based on the sensing result of the sensor unit 110.

When the removal from the body has not been detected (No at step S109), the terminal device 10 returns to step S108.

On the other hand, when the removal from the body has been detected (Yes at step S109), the terminal device 10 stops the authentication function (step S110) and ends the processing. Here, the terminal device 10 stops the authentication function by executing the unrecoverable stop processing.

The terminal device 10 executes the detection of the removal from the body at a constant cycle, for example. Accordingly, the terminal device 10 can further reduce power consumption resulting from the detection of the removal from the body.

### 3.3 Second Processing Example

Next, second processing executed by the terminal device 10 when the authentication stop processing is the recoverable stop processing will be described.

Fig. 13 is a flowchart illustrating an example of a flow of the second processing according to the embodiment of the present disclosure. The processing up to step S109 is the same as the first processing illustrated in Fig. 12, and a description thereof is thus omitted.

When the removal from the body is detected at step S109, the terminal device 10 stops the authentication function (step S501). The terminal device 10 stops the authentication function by executing the recoverable stop processing.

The terminal device 10 determines whether or not a reset instruction has been received from the communication device 20 (step S502).

When the reset instruction has been received (Yes at step S502), the terminal device 10 executes the reset processing and resets the setting of the terminal device 10 (step S503). For example, the terminal device 10 deletes the device ID, the original user information, the sensing result, and the like. Prior to executing the reset processing, the terminal device 10 may request the communication device 20 to perform the authentication (for example, the input of the master password) for executing the reset processing.

On the other hand, when the reset instruction has not been received (No at step S502), the terminal device 10 determines whether or not a re-start condition is satisfied (step S504). For example, when the terminal device 10 detects the insertion of the terminal device 10 into the body, the terminal device 10 determines that the re-start condition is satisfied.

When the re-start condition is not satisfied (No at step S504), the terminal device 10 returns to step S502.

On the other hand, when the re-start condition is satisfied (Yes at step S504), the terminal device 10 executes the original user authentication processing (step S505). For example, the terminal device 10 executes the original user authentication processing illustrated in Fig. 10.

The terminal device 10 determines whether or not the terminal device 10 is inserted into the user U_A, in accordance with the result of the original user authentication processing (step S506).

When the original user authentication has failed and it is determined that the terminal device 10 is not inserted into the user U_A, that is, the terminal device 10 is inserted into the user U_B other than the user U_A (No at step S506), the terminal device 10 returns to step S502. Alternatively, the terminal device 10 may execute the unrecoverable stop processing and end the processing.

On the other hand, when the original user authentication is successful, and it is determined that the terminal device 10 is inserted into the user U_A, (Yes at step S506), the terminal device 10 executes the re-start processing (step S507) and returns to step S106.

As described above, the terminal device 10 according to the present embodiment includes the ID authentication unit 163, the communication control unit 164, the detection unit 162, and the stop processing unit 165. The ID authentication unit 163 performs the authentication using the identification information (device ID) that identifies the terminal device 10. The communication control unit 164 communicates with the communication device 20 when the authentication by the ID authentication unit 163 is successful.

The detection unit 162 detects the removal of the terminal device 10 inserted into the body to the outside of the body. The stop processing unit 165 stops the authentication by the ID authentication unit 163 when the removal from the body is detected.

Accordingly, the terminal device 10 can prevent the unauthorized use by the person other than the user U_A.

### 4. Other Embodiments

The processing according to the above-described embodiment may be carried out in various different forms other than the above-described embodiment.

For example, in the above embodiment, when the terminal device 10 is removed from the body, the authentication stop processing is executed. However, the authentication stop processing need not necessarily be performed even when the terminal device 10 is removed. For example, the terminal device 10 need not necessarily perform the authentication stop processing when the terminal device 10 is removed in accordance with a prescribed procedure, such as when a predetermined signal is received from the communication device 20, or when the terminal device 10 is removed in a specific environmental state (electromagnetic field, temperature, pressure, or the like) .

Accordingly, the terminal device 10 can operate even outside the body by being removed using a correct (prescribed) procedure.

The terminal device 10 may include a real-time clock. The terminal device 10 may record data, such as an operation history, in the storage unit 150 together with the time of the real-time clock so that past data cannot be deleted. This can further improve the security of the terminal device 10.

In the above embodiment, the terminal device 10 periodically performs the sensing (sensing processing) of the biological information, but the terminal device 10 need not necessarily perform the sensing of the biological information other than for the original user authentication. In this case, the terminal device 10 may be used for processing including exchanges with the communication device 20, such as the electronic payment and the original user identification.

Among each of the processing steps described in the above embodiment, all or some of the processing steps described as being automatically performed can be manually performed, or all or some of the processing steps described as being manually performed can be automatically performed by a known method. In addition, processing procedures, specific names, and information including various data and parameters described in the above document and drawings can be arbitrarily changed, unless otherwise specified. For example, the various types of information illustrated in the drawings are not limited to the illustrated information.

Each of the components of each of the devices illustrated in the drawings is functionally conceptual and is not necessarily physically configured as illustrated in the drawings. In other words, specific forms of distribution and integration of each of the devices are not limited to those illustrated in the drawings, and all or some of the devices may be configured to be functionally or physically distributed or integrated in any unit, according to various loads, usage conditions, and the like. For example, the processing executed by each of the functional units (the power source management unit 220 and the control unit 240) of the communication device 20 according to the present disclosure may be executed by a dedicated device, such as a charging device.

The embodiments described above can be combined as appropriate as long as processing content is not caused to be contradictory.

In addition, the effects described in the present specification are merely examples and are not limited, and other effects may be obtained.

### 5. Supplement

Note that the present technology can also have the following configurations.
(1) A terminal device including
   an authentication unit configured to execute authentication processing for communicating with another device,
   a communication unit configured to communicate with the other device when the authentication processing is successful,
   a detection unit configured to detect removal of the terminal device, inserted into a body, to the outside of the body, and
   a stop processing unit configured to stop the authentication processing by the authentication unit when the removal to the outside of the body is detected.
(2) The terminal device according to (1), further including a re-start processing unit configured to re-start the authentication processing by the authentication unit subsequent to stopping of the authentication processing.
(3) The terminal device according to (2), in which the re-start processing unit re-starts the authentication processing in accordance with an instruction from an external device.
(4) The terminal device according to (3), in which the re-start processing unit re-starts the authentication processing using, as the instruction from the external device, at least one of an input of a password, or satisfaction of a specific environmental condition.
(5) The terminal device according to any one of (1) to (4), in which the stop processing unit stops the authentication processing by stopping at least one of communication by the communication unit, or control by a control unit.
(6) The terminal device according to any one of (1) to (5), in which the stop processing unit stops the authentication processing to cause the authentication processing to not be re-started.
(7) The terminal device according to (6), in which the stop processing unit stops the authentication processing to cause the authentication processing to be re-started or to not be re-started, in accordance with a predetermined condition.
(8) The terminal device according to (7), in which the predetermined condition is at least one of a usage time period, a number of times of use, and an application of the terminal device, or an instruction from the other device.
(9) The terminal device according to any one of (6) to (8), in which the stop processing unit stops the authentication processing by deleting at least one of a program or data.
(10) The terminal device according to any one of (6) to (9), in which the stop processing unit stops the authentication processing by destroying at least a part of a circuit.
(11) The terminal device according to any one of (6) to (10), in which the stop processing unit stops the authentication processing by moving a state of hardware logic that performs the authentication processing to a state in which state machine transition is not possible.
(12) The terminal device according to any one of (1) to (11), further including a wearable authentication unit that performs authentication of a wearer wearing the terminal device.
(13) The terminal device according to (12), in which the wearable authentication unit performs the authentication of the wearer using at least one of a password, or biological information of the wearer.
(14) The terminal device according to (13), further including a sensing unit configured to sense the biological information of the wearer. The wearable authentication unit performs the authentication of the wearer using sensing information sensed in advance, and the biological information sensed by the sensing unit.
(15) The terminal device according to (13) or (14), in which the wearable authentication unit performs the authentication of the wearer in at least one of a case where the detection unit detects the insertion for a first time, or a case where the detection unit detects the insertion subsequent to the stop processing unit stopping the authentication.
(16) The terminal device according to any one of (1) to (15), in which the detection unit detects the insertion in accordance with at least one of an elapse of a certain period of time subsequent to a predetermined operation being performed on the terminal device, or movement of the terminal device.
(17) The terminal device according to any one of (1) to (16), further including a sensor unit configured to sense biological information of a wearer wearing the terminal device. The sensor unit senses the biological information at a predetermined cycle.
(18) The terminal device according to any one of (1) to (17), further including a sensor unit configured to sense biological information of a wearer wearing the terminal device, using a first sensor and a second sensor. The first sensor is disposed at a first end portion of the terminal device, and the second sensor is disposed at a second end portion of the terminal device, the second end portion being opposite to the first end portion.
(19) The terminal device according to any one of (1) to (18), further including a sensor unit configured to sense biological information of a wearer wearing the terminal device, using a third sensor and a fourth sensor. The third sensor is disposed at a first surface of the terminal device, and the fourth sensor is disposed at a second surface of the terminal device, the second surface being opposite to the first surface.
(20) A method including
   executing authentication processing for communicating with another device,
   communicating with the other device when the authentication processing is successful,
   detecting removal of the terminal device, inserted into a body, to the outside of the body, and
   stopping the authentication processing when the removal to the outside of the body is detected.

### Reference Signs List

10 Terminal device
20 Communication device
110 Sensor unit
120,210 Communication unit
121,141,211,221 Antenna
130 Battery unit
140,220 Power source management unit
150,230 Storage unit
160,240 Control unit
161 Sensor control unit
162 Detection unit
164 Communication control unit
165 Stop processing unit
166 Original user authentication unit
167 Re-start processing unit
168 Management unit
241 Management setting unit
243 Transmission/reception unit
244 Switching instruction unit
245 Reset instruction unit
246 Re-start instruction unit

## Claims

1. A terminal device comprising:
an authentication unit configured to execute authentication processing for communicating with another device;
a communication unit configured to communicate with the other device when the authentication processing is successful;
a detection unit configured to detect removal of the terminal device, inserted into a body, to the outside of the body; and
a stop processing unit configured to stop the authentication processing by the authentication unit when the removal to the outside of the body is detected.

2. The terminal device according to claim 1, further comprising:
a re-start processing unit configured to re-start the authentication processing by the authentication unit subsequent to stopping of the authentication processing.

3. The terminal device according to claim 2, wherein
the re-start processing unit re-starts the authentication processing in accordance with an instruction from an external device.

4. The terminal device according to claim 3, wherein
the re-start processing unit re-starts the authentication processing using, as the instruction from the external device, at least one of an input of a password, or satisfaction of a specific environmental condition.

5. The terminal device according to claim 1, wherein
the stop processing unit stops the authentication processing by stopping at least one of communication by the communication unit, or control by a control unit.

6. The terminal device according to claim 1, wherein
the stop processing unit stops the authentication processing to cause the authentication processing to not be re-started.

7. The terminal device according to claim 6, wherein
the stop processing unit stops the authentication processing to cause the authentication processing to be re-started or to not be re-started, in accordance with a predetermined condition.

8. The terminal device according to claim 7, wherein
the predetermined condition is at least one of a usage time period, a number of times of use, and an application of the terminal device, or an instruction from the other device.

9. The terminal device according to claim 6, wherein
the stop processing unit stops the authentication processing by deleting at least one of a program or data.

10. The terminal device according to claim 6, wherein
the stop processing unit stops the authentication processing by destroying at least a part of a circuit.

11. The terminal device according to claim 6, wherein
the stop processing unit stops the authentication processing by moving a state of hardware logic that performs the authentication processing to a state in which state machine transition is not possible.

12. The terminal device according to claim 1, further comprising:
a wearable authentication unit that performs authentication of a wearer wearing the terminal device.

13. The terminal device according to claim 12, wherein
the wearable authentication unit performs the authentication of the wearer using at least one of a password, or biological information of the wearer.

14. The terminal device according to claim 13, further comprising:
a sensing unit configured to sense the biological information of the wearer, wherein
the wearable authentication unit performs the authentication of the wearer using sensing information sensed in advance, and the biological information sensed by the sensing unit.

15. The terminal device according to claim 13, wherein
the wearable authentication unit performs the authentication of the wearer in at least one of a case where the detection unit detects the insertion for a first time, or a case where the detection unit detects the insertion subsequent to the stop processing unit stopping the authentication.

16. The terminal device according to claim 1, wherein
the detection unit detects the insertion in accordance with at least one of an elapse of a certain period of time subsequent to a predetermined operation being performed on the terminal device, or movement of the terminal device.

17. The terminal device according to claim 1, further comprising:
a sensor unit configured to sense biological information of a wearer wearing the terminal device, wherein
the sensor unit senses the biological information at a predetermined cycle.

18. The terminal device according to claim 1, further comprising:
a sensor unit configured to sense biological information of a wearer wearing the terminal device, using a first sensor and a second sensor, wherein
the first sensor is disposed at a first end portion of the terminal device, and
the second sensor is disposed at a second end portion of the terminal device, the second end portion being opposite to the first end portion.

19. The terminal device according to claim 1, further comprising:
a sensor unit configured to sense biological information of a wearer wearing the terminal device, using a third sensor and a fourth sensor, wherein
the third sensor is disposed at a first surface of the terminal device, and
the fourth sensor is disposed at a second surface of the terminal device, the second surface being opposite to the first surface.

20. A method comprising:
executing authentication processing for communicating with another device;
communicating with the other device when the authentication processing is successful;
detecting removal of the terminal device, inserted into a body, to the outside of the body; and
stopping the authentication processing when the removal to the outside of the body is detected.
